(19) Europäisches Patentamt
European Patent Office
Office européen des brevets

(11) **EP 3 266 473 A1**

(12) **EUROPÄISCHE PATENTANMELDUNG**

(43) Veröffentlichungstag:
**10.01.2018 Patentblatt 2018/02**

(51) Int Cl.:
***A61L 24/00*** (2006.01)          ***A61K 33/40*** (2006.01)
***C08L 33/12*** (2006.01)

(21) Anmeldenummer: **17167025.0**

(22) Anmeldetag: **19.04.2017**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**MA MD**

(30) Priorität: **04.07.2016 DE 102016212091**

(71) Anmelder: **Heraeus Medical GmbH
61273 Wehrheim (DE)**

(72) Erfinder:
• **VOGT, Sebastian
99092 Erfurt (DE)**
• **CALDERÓN ORTIZ, Lorena
60437 Frankfurt (DE)**

(74) Vertreter: **Heraeus IP
Heraeus Holding GmbH
Schutzrechte
Heraeusstraße 12-14
63450 Hanau (DE)**

(54) **ANTISEPTISCHER POLYMETHYLMETHACRYLAT-KNOCHENZEMENT**

(57) Es wird eine antiseptische Zusammensetzung zur Verwendung als Knochenzement, insbesondere ein antiseptischer Polymethylmethacrylat-Knochenzement vorgeschlagen. Die Zusammensatzung ist härtbar und weist ein pharmakologisch verträgliches Salz einer Monoperoxydicarbonsäure auf, wobei das Salz der Monoperoxydicarbonsäure in Gegenwart von Wasser aus der Zusammensetzung herauslösbar ist. Vorzugsweise wird das Salz der Monoperoxydicarbonsäure in der Zusammensetzung als Pulver eingesetzt, wobei das Pulver eine mittlere Partikelgröße von nicht mehr als 250 μm aufweist. Vorzugsweise wird das Salz der Monoperoxydicarbonsäure in wässriger Lösung bei Raumtemperatur innerhalb von 5 min nicht durch das Enzym Katalase zersetzt.

EP 3 266 473 A1

**Beschreibung**

Die Erfindung betrifft eine Zusammensetzung zur Verwendung als Knochenzement, insbesondere einen antiseptischen Polymethylmethacrylat-Knochenzement, wobei die Zusammensetzung härtbar ist, und die Zusammensetzung ein pharmakologisch verträgliches Salz einer Monoperoxydicarbonsäure aufweist und das Salz der Monoperoxydicarbonsäure in Gegenwart von Wasser aus der Zusammensetzung herauslösbar ist. Ferner betrifft die Erfindung einen Kit zur Verwendung als Knochenzement, insbesondere einen antiseptischen Polymethylmethacrylat-Knochenzement, wobei der Kit eine Paste A und eine Paste B aufweist, wobei mindestens eine der Pasten A und/oder B als Komponente ein pharmakologisch verträgliches Salz einer Monoperoxydicarbonsäure beinhaltet. Weiterhin betrifft die Erfindung ein Kit aufweisend eine Pulverkomponente C und eine flüssige Monomerkomponente D, wobei die Pulverkomponente C als Komponente ein pharmakologisch verträgliches Salz einer Monoperoxydicarbonsäure beinhaltet.

In der Orthopädie und Unfallchirurgie werden Polymethylmethacrylat-Knochenzemente zur dauerhaften mechanischen Fixierung von Totalgelenkendoprothesen eingesetzt. Diese basieren auf Pulver-Flüssigkeits-Systemen, wobei Methylmethacrylat üblicherweise als Monomer verwendet wird. Ein allgemeiner Überblick findet sich in: K.-D. Kühn, PMMA Cements, Springer-Verlag Berlin Heidelberg 2014.

Neben den Pulver-Flüssigkeitszementen wurden auch Polymethylmethacrylat-Knochenzemente vorgeschlagen, die auf der Verwendung von Zementpasten beruhen. DE 10 2007 052 116 A1 betrifft einen einkomponentigen Knochenzement. DE 32 45 956 A1, DE 10 2007 050 762 B3 und DE 10 2008 030 312 A1 beschreiben zweikomponentige Knochenzemente aus zwei Zementpasten, die separat in geeigneten Kartuschen gelagert werden. Diese Pasten enthalten immer ein Methacrylat-Monomer und mindestens ein darin gelöstes Polymer. Weiterhin können diese Pasten noch im Methacrylat-Monomer unlösliche, vernetzte Polymerpartikel enthalten. In den beiden pastenförmigen Komponenten sind Bestandteile von Redoxinitiatorsystemen separat enthalten, die erst nach der Vermischung der beiden pastenförmigen Komponenten unter Bildung von Radikalen reagieren, welche die radikalische Polymerisation des Methacrylat-Monomeren auslösen, die zur Aushärtung des gemischten Zementteigs führt.

Es werden gegenwärtig neben wirkstofffreien Polymethylmethacrylat-Knochenzementen hauptsächlich Antibiotika enthaltende Polymethylmethacrylat-Knochenzemente verwendet. Diese Knochenzemente enthalten die Antibiotika Gentamicinsulfat oder Tobramycinsulfat und werden vornehmlich zur mechanischen Fixierung von primären Gelenkendoprothesen verwendet. Bei antibiotischen Knochenzementen, welche für die mechanische Fixierung von Revisions-Gelenkendoprothesen vorgesehen sind, kommen Antibiotika-Kombinationen von Gentamicinsulfat und Clindamycinhydrochlorid sowie von Vancomycinhydrochlorid und Gentamicinsulfat häufig zur Anwendung.

Es gab eine Reihe von Bemühungen, Antiseptika anstelle von Antibiotika in Polymethylmethacrylat-Knochenzemente zu integrieren.

In der WO 82/01990 A1 wird ein Knochenzement vorgeschlagen, der bis zu 5 Gew.-% Silbersalze enthält. Eine antimikrobielle Zusammensetzung, die bis zu 10 Gew.-% elementares Silber und zusätzlich noch Titandioxid oder Tantaloxid enthält, wird in der US 4,849,223 A vorgeschlagen.

In der EP 1 313 518 A1 wurde ein Knochenzement beschrieben, der Silberpartikel mit einer Größe von 20 $\mu$m enthält. Diese Silberpartikel sind aus kleineren Silberpartikeln, mit einer Größe im Nanometerbereich, aufgebaut. Kritisch ist bei der Verwendung von elementaren Silber oder auch Silbersalzen, dass die eigentlich mikrobiozid wirkenden Silber-Ionen unselektiv neben mikrobiellen Strukturen auch mit Strukturen des humanen Gewebes wechselwirken. So ist die Bildung von in Wasser gering löslichen Salzen mit Cystein und Cystein enthaltenden Proteinen möglich. Weiterhin können Silber-Ionen mit Phosphat-Ionen unter Bildung des schwer löslichen Silberorthophosphats reagieren. Es ist davon auszugehen, dass in den humanen Körper eingebrachte Silberverbindungen praktisch nicht ausgeschieden werden können, wie die bereits seit langem bekannte Erscheinung der Argyrie, der irreversiblen Hautverfärbung durch Silber, zeigt. In der EP 1 648 531 A1 wurde ein Polymethylmethacrylat-Knochenzement offenbart, der kationische Antiseptika enthält, wobei besonders das Antiseptikum Polyhexamethylenbiguanid bevorzugt wird. Kritsch muss in diesem Zusammenhang diskutiert werden, dass diese kationischen Antiseptika vom humanen Gewebe nicht abgebaut werden können und die Gefahr einer lokalen Akkumulation besteht.

In der DE 10 2012 022 419 A1 wird ein antiseptischer Polymethylmethacrylat-Knochenzement vorgeschlagen, der Wasserstoffperoxid abgebende Addukte, wie Harnstoff-Peroxid, oder Wasserstoffperoxid-freigebende Salze, wie Calciumperoxid, enthält. Bei Einwirkung von Wasser oder wässrigen Lösungen auf den ausgehärteten Polymethylmethacrylat-Knochenzement wird Wasserstoffperoxid freigesetzt. Eine antiseptische Wirkung ist nur unmittelbar an der Oberfläche des Knochenzementes gegeben, da das freigesetzte Wasserstoffperoxid innerhalb kurzer Zeit nach Kontakt mit Körperflüssigkeit durch das Enzym Katalase (EC 1.11.1.6) zersetzt wird.

Der Erfindung liegt die Aufgabe zu Grunde eine Zusammensetzung zur Verwendung als Knochenzement, insbesondere als Polymethacrylat-Knochenzement, anzugeben, die nach einer Implantation durch Einwirkung von Körperflüssigkeiten ein breit wirksames Antiseptikum an der Oberfläche des Knochenzements freisetzt, wobei das Antiseptikum temporär eine mikrobielle Besiedlung der Zementoberfläche verhindern soll. Eine weitere Aufgabe der Erfindung ist es daher, eine Zusammensetzung zur Verwendung als Knochenzement, insbesondere als Polymethacrylat-Knochenzement, anzugeben, die ein Antiseptikum bereitstellt das nicht durch das Enzym Katalase innerhalb weniger Minuten zersetzt wird. Eine weitere Aufgabe der Erfindung ist es eine Zusammensetzung zur Verwendung als Knochenzement, insbesondere als Polymethacrylat-Knochenzement, anzugeben, die ein Antiseptikum bereitstellt das die radikalische Polymerisation während der Aushärtung des Knochenzements nicht stört. Der antiseptische Knochenzement muss die Anforderungen der ISO5833 hinsichtlich der Druckfestigkeit von $\geq$ 70 MPa, der Biegefestigkeit von $\geq$ 50 MPa und des Biegemoduls von $\geq$ 1800 MPa erfüllen. Eine weitere Aufgabe der Erfindung ist es eine Zusammensetzung zur Verwendung als Knochenzement, insbesondere als Polymethacrylat-Knochenzement, anzugeben, die gekennzeichnet ist durch eine Kombination der vorgenannten Merkmale.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen.

Einen Beitrag zur Erfüllung mindestens einer der erfindungsgemäßen Aufgaben leistet eine Ausführungsform 1 einer Zusammensetzung zur Verwendung als Knochenzement, wobei die Zusammensetzung härtbar ist, und die Zusammensetzung ein pharmakologisch verträgliches Salz einer Monoperoxydicarbonsäure aufweist und das Salz der Monoperoxydicarbonsäure in Gegenwart von Wasser aus der Zusammensetzung herauslösbar ist.

Es wurde überraschend gefunden, dass pharmakologisch verträgliche Salze von Monoperoxydicarbonsäuren, die aus ausgehärteten Knochenzementen, insbesondere Polymethylmethacrylat-Knochenzementen, durch Einwirkung von wässrigen Lösungen herausgelöst werden oder durch letztere herauslösbar sind, eine lang anhaltende antiseptische Wirkung an der Zementoberfläche zeigen.

Erfindungsgemäß ist die Monoperoxydicarbonsäure aus der Zusammensetzung herauslösbar, vorzugsweise nach einer Härtung, wie durch Polymerisation eines flüssigen Monomers und mindestens einem in dem Monomer löslichen organischen Polymer.

Freie Monoperoxydicarbonsäuren sind in der Regel instabile Verbindungen, die im Rahmen der Erfindung aufgrund dessen in Form stabiler und pharmakologisch verträglicher beziehungsweise pharmakologisch akzeptabler Metallsalze eingesetzt werden. Beispiele für pharmakologisch verträgliche und/oder akzeptable Salze sind Alkalimetallsalze, insbesondere Natrium- Kalium- und Lithiumsalze. Ferner sind dies die Salze der Erdalkalimetalle, insbesondere Magnesium- und Calcium-Salze und die Salze einiger Übergangsmetalle, insbesondere Eisen-, Zink- und Kupfer-Salze.

Unter einer Monoperoxydicarbonsäure wird eine Dicarbonsäure mit zwei Carboxyl-Gruppen verstanden, bei der eine der beiden Carboxyl-Gruppen oxidiert in Form einer Peroxycarboxylgruppe vorliegt.

Bevorzugt verfügt die erfindungsgemäße Monoperoxydicarbonsäure über wenigstens 4 Kohlenstoff-Atome.

Bei den Monoperoxydicarbonsäuren handelt es sich insbesondere um aliphatisch unverzweigte oder verzweigte Monoperoxydicarbonsäuren mit 4 bis 20 C-Atomen, um alicyclische Monoperoxydicarbonsäuren mit 8 bis 12 C-Atomen, um aromatische Monoperoxydicarbonsäuren mit 8 bis 12 C-Atomen oder eine Mischung umfassend mindestens zwei der genannten Monoperoxydicarbonsäuren.

Beispiele für aliphatische und alicyclische Monoperoxydicarbonsäuren sind Monoperoxybernsteinsäure, Monoperoxyglutarsäure, Monoperoxyadipinsäure, Monoperoxycyclohexyldicarbonsäure, Monoperoxypimelinsäure, Monoperoxykorksäure, Monoperoxyazelainsäure, Monoperoxysebacinsäure, Monoperoxynonandicarbonsäure, Monoperoxydecandicarbonsäure, Monoperoxyundecandicarbonsäure und Monoperoxydodecandicarbonsäure, wobei Monoperoxyglutarsäure, Monoperoxybernsteinsäure und Monoperoxycyclohexyldicarbonsäure besonders bevorzugt sind.

Beispiele für aromatische Monoperoxydicarbonsäuren sind Monoperoxyphthalsäure, Monoperoxyterephthalsäure, Monoperoxyisophthalsäure und Monoperoxynaphthalsäure, wobei Monperoxyphthalsäure besonders bevorzugt ist.

In bevorzugter Weise verfügen die pharmokologisch verträglichen oder pharmokologisch akzeptablen Salze der Monoperoxydicarbonsäuren über eine freie Säurefunktion, wobei die Carboxylgruppe als deprotoniertes Carboxylat vorliegt und die Peroxycarboxylgruppe als Peroxycarbonsäuregruppe (-COOOH) vorliegt. Die Monoperoxydicarbonsäuren bilden daher bevorzugt mit Alkalimetallen Salze mit einer Monoperoxydicarbonsäure im Salz und mit Erdalkalimetallen Erdalkalimetallsalze mit zwei Monoperoxydicarbonsäuren im Salz. Entsprechendes gilt für Übergangsmetallsalze, je nach Stellung des Übergangmetalls im Periodensystem und der bevorzugten Oxidationsstufe des pharmakologisch verträglichen und/oder pharmakologisch akzeptablen Übergangmetalls.

Die pharmakologisch verträglichen oder pharmakologisch akzeptablen Salze der Monoperoxydicarbonsäuren sind aus der erfindungsgemäßen Zusammensetzung durch Einwirkung von Wasser herauslösbar. "Herauslösbar" bedeutet in diesem Zusammenhang, dass das Monoperoxydicarbonsäure-Salz entweder teilweise herauslösbar oder vollständig herauslösbar ist, bevorzugt ist das Monoperoxydicarbonsäure-Salz vollständig aus der Zusammensetzung herauslösbar.

"In Gegenwart von Wasser" bedeutet erfindungsgemäß, dass die Zusammensetzung mit Wasser in Form einer wässrigen Lösung, einer teilwässrigen Lösung oder mit reinem Wasser in Kontakt treten kann. Zusammengefasst wird von einem wässrigen Milieu gesprochen, was bedeutet dass die Lösung Wasser enthält. Unter wässrigen Lösungen sind insbesondere salzhaltige und/oder gepufferte Lösungen, bevorzugt menschliche oder tierische Körperflüssigkeiten zu verstehen.

In einer erfindungsgemäßen Ausführungsform 2 ist die Zusammensetzung nach Ausführungsform 1 ausgestaltet, wobei die Zusammensetzung ein antiseptischer Knochenzement ist.

Monoperoxydicarbonsäuren und deren Salze setzten durch Einwirkung von Wasser oder wässrigen Lösungen, wie einem wässrigen Milieu im Körperinneren, in einer Gleichgewichtsreaktion Wasserstoffperoxid unter Bildung der entsprechenden Dicarbonsäure frei. Sowohl die Monoperoxydicarbonsäure als auch das Reaktionsprodukt Wasserstoffperoxid sind Antiseptika, die infolge ihrer oxidierenden Wirkung eine stark mikrobiozide Wirkung haben. Besonders vorteilhaft ist, dass sich Wasserstoffperoxid nicht im humanen Organismus akkumulieren kann, weil im humanen Gewebe Enzyme der Klasse der Peroxidasen (EC 1.11.1), insbesondere Katalase (EC 1.11.1.6) vorhanden sind, die Wasserstoffperoxid zersetzen. Ein weiterer Vorteil der erfindungsgemäßen Zusammensetzung oder des erfindungsgemäßen Knochenzementes ist die Vermeidung der Resistenzbildung in den Mikroorganismen, wie es bei Antibiotika basierten Therapien möglich ist. Ein weiterer Vorteil der erfindungsgemäßen Verwendung ist die lokale antiseptische Wirkung, da die vorgenannten Peroxidasen, insbesondere Katalase, das gebildete Wasserstoffperoxid wieder abbauen können.

In einer erfindungsgemäßen Ausführungsform 3 ist die Zusammensetzung nach Ausführungsform 1 oder 2 ausgestaltet, wobei die Zusammensetzung ein antiseptischer Polymethylmethacrylat-Knochenzement ist.

Unter dem Begriff Polymethylmethacrylat-Knochenzement werden konventionelle Zemente verstanden, bei denen durch Vermischung einer Polymerpulver-Komponente und einer flüssigen Monomerkomponente ein durch radikalische Polymerisation selbst aushärtender Zementteig gebildet wird. Daneben fallen unter den Begriff auch pastenförmige Polymethylmethacrylat-Knochenzemente, bei denen durch Vermischung von zwei separaten vorgequollenen Zementpasten ein selbsthärtender Zementteig gebildet wird. Exemplarisch sind dafür die Offenlegungsschriften DE 10 2007 050 762 B3, DE 10 2010 024 653 B4 und DE 10 2010 005 956 B4 genannt.

In einer erfindungsgemäßen Ausführungsform 4 ist die Zusammensetzung nach einer der Ausführungsformen 1 bis 3 ausgestaltet, wobei das Salz der Monoperoxydicarbonsäure ein Erdalkalisalz oder ein Alkalisalz ist.

Alkali- und Erdalkalisalze der Monoperoxydicarbonsäuren zeichnen sich durch eine hohe Löslichkeit und gute pharmakologische Verträglichkeit aus. Weiterhin können Alkali- und Erdalkalimetall-Salze ihre Oxidationsstufe im Gegensatz zu Salzen von Übergangsmetallen nicht wechseln. Ionen von Übergangsmetallen, zum Beispiel $Cr^{3+}$ oder $Co^{2*}$, können durch eine Änderung der Oxidationsstufe eine katalytische Zersetzung der Monoperoxydicarbonsäuren bewirken und dadurch die antiseptische Wirksamkeit beeinträchtigen.

Ferner wurde überraschend gefunden, dass Polymethylmethacrylat-Knochenzement auch die Anforderungen der ISO5833 hinsichtlich der mechanischen Eigenschaften erfüllt, wenn pharmakologisch verträgliche Salze von Monoperoxydicarbonsäuren, insbesondere Erdalkali-und Alkalisalze von Monoperoxydicarbonsäuren im Zement enthalten sind. Diese Salze haben überraschenderweise keinen störenden Einfluß auf die radikalische Polymerisation während der Aushärtung von Polymethylmethacrylat-Knochenzementen.

In einer erfindungsgemäßen Ausführungsform 5 ist die Zusammensetzung nach einer der Ausführungsformen 1 bis 4 ausgestaltet, wobei das Erdalkalisalz ein Magnesiumsalz ist.

Magesiumsalze der Monoperoxydicarbonsäuren zeichnen sich durch eine besonders hohe Löslichkeit und ausgezeichnete pharmakologische Verträglichkeit aus.

In einer erfindungsgemäßen Ausführungsform 6 ist die Zusammensetzung nach einer der Ausführungsformen 1 bis 5 ausgestaltet, wobei das Salz der Monoperoxydicarbonsäure in Methylmethacrylat bei Raumtemperatur nicht löslich ist.

In besonders bevorzugter Form sind die eingesetzten Salze der Monoperoxydicarbonsäuren in Methylmethacrylat bei 23 °C nicht löslich. Durch die geringe Löslichkeit wird zusätzlich sichergestellt, dass die eingesetzte Monoperoxydicarbonsäure oder das pharmakologisch verträgliche Salz keine unerwünschten Reaktionen mit den Bestandteilen der zu polymerisierenden Monomerkomponente des Knochenzements eingeht. Dadurch bleibt die antiseptische Wirkung der Monoperoxydicarbonsäure auch bei längeren Lagerungszeiten der Monomerkomponente des Knochenzements erhalten.

Als "nicht löslich" gelten die Salze der Monoperoxydicarbonsäuren in Methylmethacrylat dann, wenn die Löslichkeit bei 23 °C kleiner oder gleich 3 g Salz pro Liter fertige Lösung (3 g/l) ist.

In einer erfindungsgemäßen Ausführungsform 7 ist die Zusammensetzung nach einer der Ausführungsformen 1 bis 6 ausgestaltet, wobei das Salz der Monoperoxydicarbonsäure in der Zusammensetzung als Pulver eingesetzt wird, wobei das Pulver eine mittlere Partikelgröße von nicht mehr als 250 $\mu$m aufweist.

Bevorzugt werden Pulver mit einer mittleren Partikelgröße in einem Bereich von 100 $\mu$m bis 250 $\mu$m eingesetzt. Bei Pulvern mit Partikelgrößen im genannten Bereich ist die Geschwindigkeit der Freisetzung des Monoperoxydicarbonsäure-Salzes aus der Zusammensetzung, insbesondere dem Knochenzement, bei Kontakt mit einem wässrigen Milieu optimal und die mechanische Festigkeit des ausgehärteten Zements wird nur in minimalem Umfang oder nicht negativ beeinträchtigt.

Es wurde überraschend gefunden, dass Monoperoxydicarbonsäure-Salze mit Partikelgrößen von nicht mehr als 250 $\mu$m die mechanischen Eigenschaften der ausgehärteten Zusammensetzung, insbesondere einem Knochenzement, nicht negativ beeinflussen. Insbesondere werden die Anforderungen der Norm ISO5833 in Bezug auf die Biegefestigkeit, das Biegemodul und die Druckfestigkeit erfüllt

Weiterhin bedeutet eine kleine mittlere Partikelgröße des eingesetzten Pulvers, d.h. eine große spezifische Oberfläche dass das in der Monomerkomponente unlösliche Monoperoxydicarbonsäure-Salz bei Kontakt mit einem wässrigen Milieu rasch in Lösung geht. Dadurch kann das Monoperoxydicarbonsäure-Salz auch seine antiseptische Wirkung entsprechend rasch entfalten. Dies ist im Fall der Anwendung als Knochenzement besonders von Bedeutung. Denn trotz moderner Hygiene und OP-Techniken kommt es in gewissem Umfang zu Infektionen am Knochen- bzw. Weichgewebe, welches die Gelenkendoprothese umgibt. Dabei werden Früh- und Spätinfektionen unterschieden. Die Frühinfektionen werden vielfach durch Keime verursacht, die während der Implantation der Gelenkendoprothese in das humane Gewebe gelangt sind. Während dieser Phase der Behandlung ist es daher von entscheidender Bedeutung, dass das antiseptische Agens seine Wirkung rasch entfalten kann. Bevorzugt werden daher pulverförmige oder partikuläre Monoperoxydicarbonsäure-Salze mit möglichst kleinen Partikelgrößen.

In einer erfindungsgemäßen Ausführungsform 8 ist die Zusammensetzung nach einer der Ausführungsformen 1 bis 7 ausgestaltet, wobei das Salz der Monoperoxydicarbonsäure in wässriger Lösung bei Raumtemperatur innerhalb von 5 min nicht durch eine Peroxidase, bevorzugt nicht durch das Enzym Katalase zersetzt wird.

Die Monoperoxydicarbonsäuren und deren Salze werden für wenigstens 5 min nicht durch das Enzym Katalase oder durch eine andere Peroxidase abgebaut. Vorzugsweise erfolgt der Abbau nicht innerhalb von 10 min, besonders bevorzugt nicht innerhalb von 20 min. "Nicht abgebaut" bedeutet in diesem Zusammenhang, dass ab dem ersten Kontakt mit einer wässrigen oder Wasser enthaltenden Lösung oder einem wässrigen Milieu, insbesondere Körperflüssigkeit, nach der angegebenen Zeitspanne noch wenigstens 80 % der ursprünglich eingesetzten Monoperoxydicarbonsäure-Verbindung nachweisbar sind.

Erfindungsgemäß wird unter dem Begriff "Raumtemperatur" eine Temperatur von 23 °C verstanden. Die Monoperoxydicarbonsäure-Salze werden somit bei 23 °C innerhalb von 5 min nicht durch eine Peroxidase, bevorzugt nicht durch Enzym Katalyse, abgebaut. Dem Fachmann ist bekannt, dass Enzyme bei höheren Temperaturen üblicherweise über eine höhere Aktivität verfügen und bei niedrigeren Temperaturen über eine entsprechend niedrigere Aktivität. Der Abbau des Monoperoxydicarbonsäure-Salzes in der Zusammensetzung kann daher nach Kontakt mit einem wässrigen Milieu bei Temperaturen oberhalb Raumtemperatur schneller erfolgen, bei Temperaturen unterhalb Raumtemperatur entsprechend langsamer.

Im Gegensatz zu Wasserstoffperoxid, Wasserstoffperoxid-Addukten oder vergleichbaren antiseptischen Wasserstoffperoxid-Verbindungen erfolgt der Abbau von Monoperoxydicarbonsäuren oder deren Salzen im Gewebe durch Peroxidasen, insbesondere Katalase überraschenderweise mit einer signifikanten zeitlichen Verzögerung. Vermutet wird das Monoperoxydicarbonsäuren durch Katalase oder andere Peroxidasen nicht unmittelbar abgebaut werden, da diese Verbindungen nicht die entsprechende Substratstruktur aufweisen, um eine direkte Wechselwirkung mit dem aktiven Zentrum des jeweiligen Enzyms einzugehen. Weiterhin wird vermutet, dass die Monoperoxydicarbonsäure bei Kontakt mit einem wässrigen Milieu in einer langsamen Hydrolyse-Reaktion zu Wasserstoffperoxid und der entsprechenden Dicarbonsäure zersetzt wird. Erst das Hydrolyse-Produkt Wasserstoffperoxid ist der Abbaureaktion durch Katalase zugänglich. Daher wird vermutet, dass im Vergleich zu vorgenannten Wasserstoffperoxid-Verbindungen eine länger anhaltende und dadurch verbesserte antiseptische Wirksamkeit durch die erfindungsgemäßen Monoperoxydicarbonsäuren oder deren pharmakologisch verträglichen Salze erzielt wird. Als antiseptische Agentien wirken dabei vermutlich sowohl die eingesetzte Monoperoxydicarbonsäure-Verbindung als auch das Hydrolyseprodukt Wasserstoffperoxid.

In einer erfindungsgemäßen Ausführungsform 9 ist die Zusammensetzung nach einer der Ausführungsformen 1 bis 8 ausgestaltet, wobei das Salz der Monoperoxydicarbonsäure zu 0,5 Gew.-% bis 6,0 Gew.-% bezogen auf die Gesamtmenge der Zusammensetzung enthalten ist.

Zur Einstellung der gewünschten herauslösbaren Menge Monoperoxydicarbonsäure kann in der Zusammensetzung, insbesondere in dem Knochenzement, das Monoperoxydicarbonsäure-Salz in einem Anteil von 0,5 bis 6,0 Gew.-%, insbesondere von 1,5 bis 5 Gew.-%, bevorzugt von 2 bis 4,5 Gew.-%, weiter bevorzugt von 3,0 bis 4,0 Gew.-% bezogen auf die Gesamtmenge der Zusammensetzung enthalten sein. Dabei kann die Zusammensetzung auch durch Mischen von zwei oder mehr Pasten oder einer Paste und einer Pulverkomponente hergestellt werden, wobei das Monoperoxydicarbonsäure-Salz in einer oder beiden Pasten sowie auch in der Pulverzusammensetzung enthalten sein kann. Dabei ist es bevorzugt, wenn das Monoperoxydicarbonsäure-Salz mit einem Gehalt in der Zusammensetzung enthalten ist, der nach einer Aushärtung einem Gehalt von 0,5 bis 6 Gew.-% entspricht.

In einer erfindungsgemäßen Ausführungsform 10 ist die Zusammensetzung nach einer der Ausführungsformen 1 bis 9 ausgestaltet, wobei die Monoperoxydicarbonsäure ausgewählt ist aus mindestens einem Element der Gruppe Monoperoxyphthalsäure, Monoperoxyglutarsäure, Monoperoxybernsteinsäure und Monoperoxycyclohexyldicarbonsäure.

Die Monoperoxodicarbonsäure wird vorzugsweise aus der Gruppe Monoperoxyphthalsäure, Monoperoxyglutarsäure, Monoperoxybernsteinsäure und Monoperoxycyclohexyldicarbonsäure oder einer Mischung aus wenigstens zwei der vorgenannten Monoperoxydicarbonsäuren ausgewählt.

In einer erfindungsgemäßen Ausführungsform 11 ist die Zusammensetzung nach Ausführungsform 10 ausgestaltet, wobei die Monoperoxydicarbonsäure Monoperoxyphthalsäure ist.

In einer erfindungsgemäßen Ausführungsform 12 ist die Zusammensetzung nach einer der Ausführungsformen 10 oder 11 ausgestaltet, wobei die Zusammensetzung das Magnesiumsalz der Monoperoxyphthalsäure aufweist.

Vorzugsweise wird für die Zusammensetzung als Monoperoxydicarbonsäure Phthalsäure ausgewählt und in besonders bevorzugter Weise das Magnesiumsalz davon. Unter "Phthalsäure" wird *ortho*-Phthalsäure oder 1,2-Benzoldicarbonsäure verstanden.

Die Salze von aromatischen Monoperoxydicarbonsäuren sind stabiler als die Salze von aliphatischen oder alicyclischen Monoperoxydicarbonsäuren. Die Salze unsubstituierter aromatischer Peroxycarbonsäuren sind weiterhin weniger stabil als Phthalsäure, da letztere über einen elektronenziehenden Substituenten verfügt welcher die Peroxycarboxylfunktion stabilisiert. Dadurch zerfällt Monoperoxyphthalsäure weniger leicht unter Freisetzung von Sauerstoff zu Phthalsäure. Das Magnesiumsalz der Monoperoxyphthalsäure ist lagerstabil und in kostengünstig in technisch relevanten Mengen verfügbar.

In einer erfindungsgemäßen Ausführungsform 13 ist die Zusammensetzung nach einer der Ausführungsformen 1 bis 12 ausgestaltet, wobei die Zusammensetzung mindestens ein radikalisch polymerisierbares Monomer und mindestens ein organisches Polymer umfasst, wobei das Polymer in dem Monomer löslich ist.

Als in dem mindestens einen radikalisch polymerisierbaren Monomer lösliches Polymer wird ein Polymer verstanden, das sich in diesem polymerisierbaren Monomer zu wenigstens 10 g/l, vorzugsweise zu wenigstens 25 g/l, besonders bevorzugt zu wenigstens 50 g/l und ganz besonders bevorzugt zu wenigstens 100 g/l, löst. Das in dem polymerisierbaren Monomer lösliche Polymer kann ein Homopolymer oder ein Copolymer sein. Bei diesem löslichen Polymer handelt es sich vorzugsweise um ein Polymer mit einer gewichtsmittleren Molmasse (Mw) von wenigstens 150.000 g/mol, insbesondere mindestens 200.000 g/mol bis größer gleich 500.000 g/mol.

Die Menge des in dem radikalisch polymerisierbaren Monomer löslichen Polymers in der erfindungsgemäßen Zusammensetzung liegt üblicherweise in einem Bereich von 1 bis 85 Gew.-%, bezogen auf das Gesamtgewicht der erfindungsgemäßen Zusammensetzung. Entsprechend kann der Gehalt an Polymer jeweils unabhängig in einer der nachstehenden Pasten A und/oder B sowie der Pulverkomponente C und/oder der Monomerkomponente D von 1 bis 85 Gew.-% in Bezug auf die jeweilige Gesamtzusammensetzung an Paste, Pulverkomponente oder Monomerkomponente betragen.

In einer erfindungsgemäßen Ausführungsform 14 ist die Zusammensetzung nach einer der Ausführungsformen 1 bis 13 ausgestaltet, wobei das organische Polymer ausgewählt ist aus Poly(alkyl-2-acrylsäurealkylester), Poly(aryl-2-acrylsäurealkylester), Poly(arylalkyl-2-acrylsäurealkylester) jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Polymere.

Die erfindungsgemäßen Zusammensetzungen sind Knochenzemente umfassend mindestens ein organisches Polymer oder Mischungen von organischen Polymeren, die in den Monomeren löslich sind, wobei die Polymere Polyacrylate sind. Insbesondere ist das organische Polymer ausgewählt aus Poly(alkyl-2-acrylsäurealkylester), Poly(aryl-2-acrylsäurealkylester), Poly(arylalkyl-2-acrylsäurealkylester) jeweils unabhängig mit 1 bis 20 C-Atomen, vorzugsweise 1 bis 18 C-Atomen in der Alkyl-Gruppe, insbesondere mit 1 bis 4 C-Atomen, jeweils unabhängig mit 6 bis 13 C-Atomen in der Aryl-Gruppe, insbesondere mit 6, 10, 12 oder 13 C-Atomen, jeweils unabhängig mit 6 bis 14 C-Atomen in der Arylalkyl-Gruppe, insbesondere mit 8 bis 12 C-Atomen, und jeweils unabhängig mit 1 bis 10 C-Atomen in der Alkylester-Gruppen, insbesondere mit 1 bis 4 C-Atomen, oder eine Mischung umfassend mindestens zwei der genannten Polymere.

In einer erfindungsgemäßen Ausführungsform 15 ist die Zusammensetzung nach einer der Ausführungsformen 1 bis 14 ausgestaltet, wobei das organische Polymer ausgewählt ist aus der Gruppe Poly(methacrylsäuremethylester), Poly(methacrylsäureethylester), Poly(methylmethacrylsäurepropylester), Poly(methacrylsäureisopropylester), Poly(methylmethacrylat-co-methylacrylat), Poly(styren-co-methylmethacrylat), Copolymeren der genannten Verbindungen und einer Mischung umfassend mindestens zwei dieser Polymere, wobei Polymethylmethacrylat (PMMA) besonders bevorzugt eingesetzt wird.

Die Menge des in dem radikalisch polymerisierbaren Monomer löslichen Polymers in der erfindungsgemäßen Zusammensetzung liegt üblicherweise in einem Bereich von 1 bis 85 Gew.-%, bezogen auf das Gesamtgewicht der erfindungsgemäßen Zusammensetzung. Entsprechend kann der Gehalt an Polymer jeweils unabhängig in einer der nachstehenden Pasten A und/oder B sowie der Pulverkomponente C und/oder der Monomerkomponente D von 1 bis 85 Gew.-% in Bezug auf die jeweilige Gesamtzusammensetzung an Paste, Pulverkomponente oder Monomerkomponente betragen.

Als besonders bevorzugtes organisches Polymer wird mindestens ein Poly(methacrylsäuremethylester), (PMMA) und als Monomer Methacrylsäuremethylester (MMA) eingesetzt, wobei auch Gemische dieser mit weiteren Monomeren oder ein Co-Polymer von PMMA eingesetzt werden können.

Als in den Monomeren lösliche Polymere, insbesondere Polyacrylate, werden Polymere mit einem Molekulargewicht (Mw) von vorzugsweise größer gleich 200.000 g/mol zur Herstellung von Pulverkomponenten eingesetzt, bevorzugt sind Molekulargewichte von größer gleich 500.000 g/mol. Zur Verwendung in Pasten können auch Polymere mit einem Molekulargewicht von kleiner gleich 500.000 g/mol eingesetzt werden. Dabei bestimmt sich das geeignete Molekulargewicht einerseits danach, ob eine Paste oder einen Pulverkomponenten hergestellt wird sowie den weiteren Komponenten in der Paste als auch, dadurch dass das Polymer in dem eingesetzten Monomer löslich sein muss.

In einer erfindungsgemäßen Ausführungsform 16 ist die Zusammensetzung nach einer der Ausführungsformen 1 bis 15 ausgestaltet, wobei das Monomer ausgewählt ist aus mindestens einem 2-Alkyl-acrylsäurealkylester, 2-Aryl-acrylsäurealkylester, 2-Arylalkyl-acrylsäurealkylester, jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Monomere.

Dabei kann die Alkylester-Gruppe eine lineare, verzweigte oder cyclische Alkyl-Gruppe aufweisen kann, insbesondere mit 1 bis 4 C-Atomen.

Dabei sind Methacrylsäuremethylester, ein Methacrylsäureester oder ein Alkylacrylsäuremethylester bevorzugt. Besonders bevorzugt ist Methacrylsäuremethylester, wie ein Methacrylat-Monomer, insbesondere ein Methacrylat-Monomer, welches bei einer Temperatur von 25°C und einem Druck von 1.013 hPa flüssig ist. Vorzugsweise handelt es sich bei dem radikalisch polymerisierbaren Monomer nicht um einen von Bisphenol A abgeleiteten Methacrylsäureester.

Das Methacrylat-Monomer ist vorzugsweise ein Methacrylsäureester. Vorzugsweise handelt es sich bei dem Methacrylsäureester um einen monofunktionellen Methacrylsäureester. Dieser ist vorzugsweise hydrophob. Durch die Verwendung von hydrophoben monofunktionellen Methacrylsäureestern kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Das erfindungsgemäß eingesetzte radikalisch polymerisierbare Monomer weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer zeichnet sich vorzugsweise dadurch aus, dass eine wässrige Lösung, die vorzugsweise 1 : 1, mit dem radikalisch polymerisierbaren Monomer versetzt wurde, einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Gemäß einer besonders bevorzugten Ausführungsform handelt es sich bei dem Methacrylat-Monomer um Methacrylsäuremethylester, Methacrylsäureethylester oder um eine Mischung aus diesen beiden Monomeren.

In einer erfindungsgemäßen Ausführungsform 17 ist die Zusammensetzung nach einer der Ausführungsformen 1 bis 16 ausgestaltet, wobei das organische Polymer mindestens ein Poly(methacrylsäuremethylester), (PMMA) oder ein Poly(methacrylsäuremethylester-copolymer) und als Monomer Methacrylsäuremethylester (MMA) umfasst. Dabei können auch Gemische dieser mit weiteren Monomeren eingesetzt werden.

Ebenfalls Gegenstand der Erfindung sind Zusammensetzungen umfassend ein partikuläres, anorganisches Additiv, insbesondere mit einer BET-Oberfläche von mindestens 40 m$^2$/g, wobei vorzugsweise das Additiv kovalent gebundene Hydroxylgruppen aufweist. Erfindungsgemäß geeignete partikuläre anorganische Additive weisen an den Partikeln kovalent gebundene HO-Si-Gruppen (Silanol-Gruppen) auf. Durch diese an der Partikeloberfläche angeordneten Hydroxylgruppen können sich Wasserstoffbrückenbindungen zwischen den Füllstoffpartikeln ausbilden, die durch Einwirkung von mechanischer oder thermischer Energie reversibel gelöst werden können.

Das partikuläre anorganische Additiv ist bevorzugt ausgewählt aus der Gruppe aus pyrogenem Siliziumdioxid, pyrogenem Metall-Siliziummischoxide, Bentonit, Montmorillonit und einer Mischung enthaltend mindestens zwei der genannten Additive.

Daneben ist es auch möglich, hydrophobiertes pyrogenes Siliziumdioxid einzusetzen. Die Herstellung des hydrophoben Siliziumdioxids kann nach dem Stand der Technik durch Behandlung von pyrogenem Siliziumdioxid mit Dialkyldichlorsilanen (z. B. Dimethyldichlorsilan) erfolgen.

Ein ganz besonders bevorzugter partikulärer, anorganisches Additiv ist pyrogenes Siliziumdioxid mit einer BET-Oberfläche von mindestens 40 m/g, weiter bevorzugt von 200 m$^2$/g und am meisten bevorzugt von 300 m$^2$/g. Derartiges pyrogenes Siliziumdioxid ist unter anderem unter dem Markennamen Aerosil® mit spezifischen BET-Oberflächen von 50 m$^2$/g, 90 m$^2$/g, 200 m$^2$/g und 380 m$^2$/g kommerziell verfügbar.

Als partikuläres anorganisches Additiv ist pyrogenes Siliziumdioxid mit einer BET-Oberfläche von mindestens

200 m$^2$/g bevorzugt. Ebenfalls bevorzugt kann als ein partikuläres anorganisches Additiv pyrogenes Siliziumdioxid mit einer BET-Oberfläche von mindestens 300 m$^2$/g eingesetzt werden. Die erfindungsgemäß geeigneten partikulären anorganischen Additive weisen vorzugsweise Primärteilchen von etwa 7 nm und eine spezifische Oberfläche 270 bis 330 m$^2$/g auf.

Die BET-Messung ist ein Analyseverfahren zur Charakterisierung von Oberflächen von Festkörpern mit Hilfe der Gasadsorption. Die Bestimmungsmethode ist in der DIN ISO 9277:2003-05 (Bestimmung der spezifischen Oberfläche von Feststoffen durch Gasadsorption nach dem BET-Verfahren) beschrieben.

Eine erfindungsgemäße Zusammensetzung umfasst neben dem löslichen organischen Polymer, insbesondere Polymethylmethacrylat (PMMA), und dem radikalisch polymerisierbaren Monomer, insbesondere Methacrylsäuremethylester, ein partikuläres, anorganisches Additiv, vorzugsweise in einer Konzentration von 0,01 bis 0,5 Gew.-%, insbesondere von 0,01 bis 0,25 Gew.-%, bevorzugt von 0,02-0,14 Gew.-% in Bezug auf die Gesamtzusammensetzung. Erfindungsgemäß umfasst der aus der Pulverkomponente und der flüssigen Monomerkomponente gemischte Zementteig das partikuläre, anorganische Additiv in einer Konzentration von 0,02-0,14 Gew.-%.

Zusätzlich zu den vorgenannten Komponenten umfasst eine erfindungsgemäße Zusammensetzung einen Röntgenopaker, einen Polymerisationsinitiator und/oder einen Polymerisationsbeschleuniger sowie optional zusätzlich Füllstoffe, die nicht dem Additiv entsprechen und lediglich verdickende Wirkung zeigen, wie beispielsweise Siliciumdioxid mit einer BET-Oberfläche von deutlich kleiner als 35 m$^2$/g.

Einen Beitrag zur Erfüllung mindestens einer der erfindungsgemäßen Aufgaben leistet ein Kit 1 aufweisend eine Paste A und eine Paste B, wobei

(a) Paste A

(a1) mindestens ein radikalisch polymerisierbares Monomer, insbesondere zu 15 bis 85 Gew.-%, vorzugsweise 20 bis 70 Gew.-%, bevorzugt 25 bis 60 Gew.-%, besonders bevorzugt 25 bis 50 Gew.-%,

(a2) mindestens ein in (a1) lösliches organisches Polymer, insbesondere zu 5 bis 50 Gew.-%, bevorzugt 10 bis 40 Gew.-%, besonders bevorzugt 20 bis 30 Gew.-%, und

(a3) optional mindestens einen Polymerisationsinhibitor, insbesondere zu 0,05 bis 1,0 Gew.-% und

(a4) mindestens eine Komponente eines Redoxinitiatorsystems beinhaltet, insbesondere zu 0,1 bis 10 Gew.-%, bevorzugt 0,01 bis 8 Gew.-%, besonders bevorzugt 0,01 bis 5 Gew.-%, sowie optional weitere Bestandteile enthält, wie Röntgenopaker und/oder in (a1) unlöslichen Füllstoff, jeweils bezogen auf das Gesamtgewicht der Paste A, und die

(b) Paste B

(b1) mindestens ein radikalisch polymerisierbares Monomer, insbesondere zu 15 bis 85 Gew.-%, vorzugsweise 20 bis 70 Gew.-%, bevorzugt 25 bis 60 Gew.-%, besonders bevorzugt 25 bis 50 Gew.-%,

(b2) mindestens ein in (b1) lösliches organisches Polymer, insbesondere zu 5 bis 50 Gew.-%, bevorzugt 10 bis 40 Gew.-%, besonders bevorzugt 20 bis 30 Gew.-%, und

(b3) mindestens einen Polymerisationsbeschleuniger beinhaltet, insbesondere zu 0,0005 bis 0,5 Gew.-%, sowie optional weitere Bestandteile enthält, wie Röntgenopaker und/oder in (b1) unlöslichen Füllstoff, jeweils bezogen auf das Gesamtgewicht der Paste B, und wobei mindestens eine der Pasten A oder B als Komponente (a5) oder (b4) oder beide Pasten A und B als Komponenten (a5) und (b4) mindestens einen Gehalt eines pharmakologisch verträglichen Salzes einer Monoperoxydicarbonsäure beinhaltet, insbesondere zu 0,5 bis 6,0 Gew.-%, bevorzugt 1,5 bis 5 Gew.-%, besonders bevorzugt von 2 bis 4,5 Gew.-%, weiter bevorzugt zwischen 3,0 bis 4,0 Gew.-% bezogen auf die Gesamtmenge der Zusammensetzung.

Dabei kann jede der Pasten das partikuläre, anorganische Additiv in einer Konzentration 0,001 bis 2 Gew.-%, insbesondere 0,001 bis 1 Gew.-% enthalten, so dass in der durch Vermischen der Pasten A und B, insbesondere im Verhältnis von annähernd 1 zu 1 mit jeweils plus/minus 0,5, erhältlichen Zusammensetzung 0,01 bis 0,5 Gew.-% Additiv, insbesondere von 0,01 bis 0,25 Gew.-%, bevorzugt von 0,02-0,14 Gew.-% in Bezug auf die Gesamtzusammensetzung vorliegen.

Entsprechendes gilt für die nachstehende Pulverkomponente C und Paste D.

Als Monomere und Polymere werden die vorstehend definierten in den Pasten A und B eingesetzt. Entsprechendes gilt für die nachstehende Pulverkomponente C und Paste D.

Einen Beitrag zur Erfüllung mindestens einer der erfindungsgemäßen Aufgaben leistet ein Kit 2 aufweisend eine Pulverkomponente C und eine flüssige Monomerkomponente D, wobei

(c) Pulverkomponente C

(c1) mindestens ein pulverförmiges Poly(meth)acrylat, insbesondere zu 1 bis 95 Gew.-%, bevorzugt bis 85 Gew.-% und besonders bevorzugt von 50 bis 80 Gew.%,

(c2) mindestens einen pulverförmigen Röntgenopaker, insbesondere zu 3 bis 60 Gew.-%, bevorzugt 3 bis 30 Gew.-%, besonders bevorzugt von 5 bis 15 Gew.% und

(c3) mindestens einen Polymerisationsinitiator, insbesondere zu 0,01 bis 10 Gew.-%, bevorzugt 0,01 bis 8 Gew.-%, besonders bevorzugt 0,01 bis 5 Gew.-%, weiter bevorzugt 0,4 bis 3,0 Gew.-%, sowie optional weitere Bestandteile, wie pulverförmigen Füllstoff, jeweils bezogen auf das Gesamtgewicht der Pulverkomponente C beinhaltet und

(d) Monomerkomponente D

(d1) wenigstens ein radikalisch polymerisierbares Monomer, insbesondere zu 80 bis 99,9995 Gew.-%, bevorzugt von 80 bis 99 Gew.%,

(d2) optional wenigstens einen Polymerisationsinhibitor, insbesondere 0,1 bis 1,5 Gew.%,

(d3) optional wenigstens ein in (d1) lösliches organisches Polymer, insbesondere bis 19 Gew.% und

(d4) wenigstens einen Polymerisationsbeschleuniger beinhaltet, insbesondere zu 0,0005 bis 1,5 Gew.-%, bevorzugt 0,1 bis 1,5 Gew.% sowie optional weitere Bestandteile, wie Röntgenopaker und/oder in (d1) unlöslichen Füllstoff und/oder Additiv, jeweils bezogen auf das Gesamtgewicht der Monomer-komponente D beinhaltet, und wobei die Pulverkomponente C als Komponente (c4) einen Gehalt eines pharmakologisch verträglichen Salzes einer Monoperoxydicarbonsäure beinhaltet, insbesondere zu 0,5 bis 6,0 Gew.-%, bevorzugt 1,5 bis 5 Gew.-%, besonders bevorzugt von 2 bis 4,5 Gew.-%, weiter bevorzugt zwischen 3,0 bis 4,0 Gew.-% bezogen auf die Gesamtmenge der Zusammensetzung.

Erfindungsgemäß wird unter Kit ein System aus wenigstens zwei Komponenten verstanden. Obwohl im Folgenden von zwei Komponenten (z. B. Paste A und Paste B) die Rede ist, kann der Kit falls notwendig auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Vorzugsweise ist der Kit als Vorrichtung zur Herstellung von Zusammensetzungen zur Verwendung als Knochenzement so ausgestaltet, dass es einen ersten Behälter und einen zweiten Behälter aufweist, wobei der erste Behälter zum Beispiel die Paste A und der zweite Behälter die Paste B enthält, wobei wenigstens einer der Behälter zu öffnen ist, um nach dem Öffnen ein Vermischen der Pasten A und B zu gestatten, und eine Mischeinheit zum Vermischen der Pasten A und B.

Im Falle von Kit 1 werden dazu die wenigstens zwei Pasten A und B miteinander vermischt, wobei die erfindungsgemäße Zusammensetzung erhalten wird. Das Mischungsverhältnis beträgt vorzugsweise 0,5 bis 1,5 Gewichtsteile an Paste A und 0,5 bis 1,5 Gewichtsteile an Paste B. Gemäß einer besonders bevorzugten Ausführungsform beträgt der Anteil der Paste A 30 bis 70 Gew.-% und der Anteil der Paste B 30 bis 70 Gew.-%, jeweils bezogen auf das Gesamtgewicht der Pasten A und B.

Nach dem Vermischen der Pasten von Kit 1 ist die letztlich erhaltene Zusammensetzung spätestens nach 1 bis 2 Minuten nach der Norm ISO 5833 klebfrei.

Falle von Kit 2 beträgt das Mischungsverhältnis der Pulverkomponente C und der Monomerkomponente D vorzugsweise 3 zu 1 bis 1 zu 2, insbesondere um 2 zu 1 Gewichtsteile. Das Mischen kann mit üblichen Mischgeräten, beispielsweise einem statischen Mischer oder einem dynamischen Mischer erfolgen. Entsprechend kann das Kit 2 als Vorrichtung zur Herstellung der erfindungsgemäßen Zusammensetzung einen ersten Behälter für die Pulverkomponente C und einen zweiten Behälter für die Monomerkomponente D aufweisen.

Dabei werden vorzugsweise als pharmakologisch verträgliche Monoperoxydicarbonsäure-Salze die vorgenannten Verbindungen in einer der Pasten A und/oder B oder alternativ der Pulverkomponenten C in den genannten Mengen jeweils bezogen auf 100 Gew.% der Gesamtzusammensetzung eingesetzt.

Als pulverförmiges Poly(meth)acrylat wird ein organisches Polymer in Form eines Pulvers gemäß vor-stehender Definition eingesetzt, bevorzugt ist pulverförmiges PMMA. Generell kann das Additiv mit einem Gehalt sowohl in der Pulverkomponente als auch in der Paste vorliegen.

Im Falle einer erfindungsgemäßen Zusammensetzung, welche durch die Kombination zweier Pasten A und B oder der Pulverkomponente C und der Monomerkomponente D eines Zweikomponenten-Systems erhalten wurde, beinhaltet diese Zusammensetzung vorzugsweise mindestens einen Polymerisationsinitiator (der in der einen Paste/Komponente des Zweikomponenten-Systems enthalten war) und wenigstens einen Polymerisationsbeschleuniger (der in der anderen Paste/Komponente des Zweikomponenten-Systems enthalten war).

Üblicherweise enthalten die Paste A und/oder B sowie die Pulverkomponente C und/oder die Monomerkomponente D jeweils unabhängig voneinander einen Röntgenopaker.

Die vorgenannten Pasten A und B können in jedem beliebigen Verhältnis miteinander gemischt werden, wobei sich die Verwendung der Pasten A und B in einem Verhältnis von im Wesentlichen 1 zu 1, die miteinander gemischt werden können als bevorzugt erwiesen hat, wobei das Verhältnis zueinander unabhängig plus/minus 50 % schwanken kann.

Die erfindungsgemäßen Zusammensetzungen, Pasten und/oder Pulverkomponenten können mindestens einen Polymerisationsinitiator (der vorzugsweise in dem radikalisch polymerisierbaren Monomer löslich ist), wenigstens einen Polymerisationsbeschleuniger (der vorzugsweise in dem radikalisch polymerisierbaren Monomer löslich ist), gegebenenfalls wenigstens einen Co-Polymerisationsbeschleuniger oder gegebenenfalls wenigstens einen Co-Polymerisationsinitiator beinhalten.

Im Falle eines Einkomponenten-Systems als erfindungsgemäße Zusammensetzung handelt es sich bei dem Polymerisationsinitiator vorzugsweise um einen aktivierbaren Polymerisationsinitiator, z. B. um einen in der Zusammensetzung, die als Paste vorliegt, gelösten oder suspendierten Photoinitiator oder um ein in der Paste gelöstes oder suspendiertes Photoiniatorsystem. Es ist auch möglich, einen Initiator oder Initiatoren dort vorzusehen, wo er bzw. sie vorübergehend zu Kontakt mit der Paste kommt, etwa in einem Behälterteil, einer Dosiervorrichtung oder einer Transportkanüle.

Auch kann im Falle eines Einkomponenten-Systems die erfindungsgemäße Zusammensetzung oder Paste neben dem aktivierbaren Polymerisationsinitiator auch einen elektrisch leitfähigen Röntgenopaker beinhalten. Hier eignen sich besonders Partikel von Kobalt, Eisen, NdFeB, SmCo, Kobalt-Chrom-Stahl, Zirkonium, Hafnium, Titan, Titan-Aluminium-Silizium-Legierungen und Titan-Niobium-Legierungen mit einer Partikelgröße von 0,5-500 $\mu$m. In dem elektrisch leitfähigen Röntgenopaker können durch magnetische Wechselfelder mit einer Frequenz im Bereich von 500 Hz bis 50 kHz Wirbelströme induziert werden, die zu einer Erwärmung des Opakers führen. Durch Wärmeübertragung wird auch der Initiator aufgeheizt und zum thermischen Zerfall gebracht.

Einen Beitrag zur Erfüllung mindestens einer der erfindungsgemäßen Aufgaben leistet eine Ausführungsform 1 eines härtbaren Knochenzements, wobei der Knochenzement erhältlich ist durch Polymerisation einer erfindungsgemäßen Zusammensetzung nach einer der Ausführungsformen 1 bis 17. Einen weiteren Beitrag zur Erfüllung mindestens einer der erfindungsgemäßen Aufgaben leistet eine Ausführungsform 2 eines härtbaren Knochenzements, wobei der Knochenzement erhältlich ist durch Vermischen der Pasten A und B von Kit 1. Einen weiteren Beitrag zur Erfüllung mindestens einer der erfindungsgemäßen Aufgaben leistet eine Ausführungsform 3 eines härtbaren Knochenzements, wobei der Knochenzement erhältlich ist durch Vermischen der Pulverkomponente C mit der Monomerkomponente D und Polymerisation von Kit 2.

Dabei weist der härtbare Knochenzement einen Gehalt an pharmakologisch verträglichem Monoperoxydicarbonsäure-Salz von insbesondere 0,5 bis 6,0 Gew.-%, bevorzugt 1,5 bis 5 Gew.-%, besonders bevorzugt von 2 bis 4,5 Gew.-% und weiter bevorzugt zwischen 3,0 bis 4,0 Gew.-% bezogen auf die Gesamtzusammensetzung auf.

Einen Beitrag zur Erfüllung mindestens einer der erfindungsgemäßen Aufgaben leistet eine Ausführungsform 1 eines gehärteten Knochenzements, erhältlich durch Polymerisation einer Zusammensetzung nach einer der Ausführungsformen 1 bis 17. Einen weiteren Beitrag zur Erfüllung mindestens einer der erfindungsgemäßen Aufgaben leistet eine Ausführungsform 2 eines gehärteten Knochenzements, erhältlich durch Vermischen und Polymerisation der Pasten A und B von Kit 1. Einen weiteren Beitrag zur Erfüllung mindestens einer der erfindungsgemäßen Aufgaben leistet eine Ausführungsform 3 eines gehärteten Knochenzements, erhältlich durch Vermischen und Polymerisation der Pulverkomponente C mit der Monomerkomponente D von Kit 2.

Dabei weist der gehärtete Knochenzement einen Gehalt an pharmakologisch verträglichem Monoperoxydicarbonsäure-Salz von insbesondere 0,5 bis 6,0 Gew.-%, bevorzugt 1,5 bis 5 Gew.-%, besonders bevorzugt von 2 bis 4,5 Gew.-% und weiter bevorzugt zwischen 3,0 bis 4,0 Gew.-% bezogen auf die Gesamtzusammensetzung auf.

Vorzugsweise wird das pharmakologisch verträgliche Monoperoxydicarbonsäure-Salz aus der härtbaren oder der gehärteten Zusammensetzung erst in Gegenwart von Wasser, feuchtem oder wässrigem Milieu herausgelöst oder ist erst dann daraus herauslösbar, besonders vorzugsweise erst nach der Aushärtung.

Einen Beitrag zur Erfüllung mindestens einer der erfindungsgemäßen Aufgaben leistet daher auch eine Ausführungsform 4 eines gehärteten Knochenzements nach einer der Ausführungsformen 1 bis 3, wobei das Salz der Monoperoxydicarbonsäure bei der Aushärtung in Gegenwart von Wasser aus der Zusammensetzung herausgelöst wird und die Monoperoxydicarbonsäure innerhalb von 5 min nicht durch eine Peroxidase, bevorzugt nicht durch das Enzym Katalase zersetzt wird.

Einen Beitrag zur Erfüllung mindestens einer der erfindungsgemäßen Aufgaben leistet auch eine Ausführungsform 1 eines Formkörpers erhältlich durch Polymerisation einer Zusammensetzung nach einer der Ausführungsformen 1 bis 17. Einen weiteren Beitrag zur Erfüllung mindestens einer der erfindungsgemäßen Aufgaben leistet auch eine Ausführungsform 2 eines Formkörpers erhältlich durch Vermischen der Pasten A und B von Kit 1 und anschließende Polymerisation. Einen weiteren Beitrag zur Erfüllung mindestens einer der erfindungsgemäßen Aufgaben leistet auch eine Ausführungsform 3 eines Formkörpers erhältlich durch Vermischen der Pulverkomponente C mit der Monomer-

komponente D von Kit 2 und anschließende Polymerisation.

Dabei weist der Formkörper einen Gehalt an pharmakologisch verträglichem Monoperoxydicarbonsäure-Salz von insbesondere 0,5 bis 6,0 Gew.-%, bevorzugt 1,5 bis 5 Gew.-%, besonders bevorzugt von 2 bis 4,5 Gew.-% und weiter bevorzugt zwischen 3,0 bis 4,0 Gew.-% bezogen auf den gesamten Formkörper auf.

Einen Beitrag zur Erfüllung mindestens einer der erfindungsgemäßen Aufgaben leistet auch eine Verwendung 1 einer Zusammensetzung nach einer der Ausführungsformen 1 bis 17 oder eine Verwendung 2 von Kit 1 oder eine Verwendung 3 von Kit 2 als Implantat, als antiseptisches Implantat, als Revisionsimplantat, zur mechanischen Fixierung primärer Totalgelenkendoprothesen, zur mechanischen Fixierung von Revisions-Totalgelenkendoprothesen, zur Augmentation von osteoporotischem Knochengewebe und ganz besonders bevorzugt für die Vertebroplastie, Kyphoplastie und die Augmentation von Bohrlöchern in osteoporotischem Knochengewebe, zur Auffüllung von Knochenkavitäten, zur Femuroplastie, zur Herstellung von Spacern, zur mechanischen Fixierung von Gelenkprothesen, zur Abdeckung von Schädeldefekten oder zur Herstellung von Trägermaterialien für die lokale Antibiotika-Therapie oder als Trägermaterial für eine lokale Freisetzung von pharmazeutisch wirksamen Substanzen.

Als Polymerisationsinitiator kommen insbesondere Peroxide und Barbitursäurederivate in Betracht, wobei die Peroxide und Barbitursäurederivate vorzugsweise in dem polymerisierbaren Monomer zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löslich sind. Die Polymerisationsinitiatoren zerfallen radikalisch, ggf. in der Regel unter Bildung von Wasserstoffradikalen und Abspaltung von Sauerstoff. In Gegenwart von Wasser bildet der Initiator kein Wasserstoffperoxid. Cumolhydroperoxid kann in Gegenwart von Wasser zu Keton und Phenol umlagern. Radikale wie H-O-O·, R-O-O·, R = organischer Rest, gelten nicht als Wasserstoffperoxid

Unter einem Peroxid werden im Sinne des Polymerisationsinitiators Verbindungen verstanden, die wenigstens eine Peroxogruppe (-O-O-) enthalten. Das Peroxid weist vorzugsweise keine freien aciden Gruppen auf. Bei dem Peroxid kann es sich um ein anorganisches Peroxid oder um ein organisches Peroxid handeln, wie zum Beispiel ein toxikologisch unbedenkliches Hydroperoxid. Das Hydroperoxid ist jedoch kein Wasserstoffperoxid. Gemäß einer besonders bevorzugten Ausführungsform ist das Peroxid aus der Gruppe ausgewählt, die aus Cumol-hydroperoxid, 1,1,3,3-Tetramethylbutyl-hydroperoxid, t-Butyl-hydroperoxid, t-Amylhydroperoxid, Di-Isopropylbenzen-mono-hydroperoxid und einer Mischung aus mindestens zwei davon besteht.

Bei dem Barbitursäurederivat handelt es sich vorzugsweise um ein Barbitursäurederivat ausgewählt aus der Gruppe bestehend aus 1-monosubstituierten Barbituraten, 5-monosubstituierten Barbituraten, 1,5-disubstituierten Barbituraten-und 1,3,5-trisubstituierten Barbituraten. Gemäß einer besonderen Ausgestaltung der erfindungsgemäßen Paste ist das Barbitursäurederivat ausgewählt aus der Gruppe bestehend aus 1,5-disubstituierten Barbituraten-und 1,3,5-trisubstituierten Barbituraten.

Die Art der Substituenten an der Barbitursäure ist nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5-disubstituierte Thiobarbiturate oder 1,3,5-trisubstituierte Thiobarbiturate bevorzugt sein. Gemäß einer bevorzugten Ausführungsform weisen die Substituenten jeweils eine Länge von 1 bis 10 Kohlenstoffatomen, mehr bevorzugt eine Länge von 1 bis 8 Kohlenstoffatomen und ganz besonders bevorzugt eine Länge im Bereich von 2 bis 7 Kohlenstoffatomen auf.

Erfindungsgemäß bevorzugt sind Barbiturate, die an Position 1 und an Position 5 jeweils einen Substituenten oder an den Positionen 1, 3 und 5 jeweils einen Substituenten tragen. Gemäß einer weiteren bevorzugten Ausführungsform ist das Barbitursäure-Derivat ein 1,5-disubstituiertes Barbiturat oder ein 1,3,5-trisubtituiertes Barbiturat. Gemäß einer besonders bevorzugten Ausführungsform ist das Barbitursäurederivat aus der Gruppe ausgewählt, die aus 1-Cyclohexyl-5-ethyl-barbitursäure, 1-Phenyl-5-ethyl-barbitursäure und 1,3,5-Trimethylbarbitursäure besteht.

Als Polymerisationsbeschleuniger sind Schwermetallverbindungen, die aus der Gruppe bestehend aus Schwermetallsalzen und Schwermetallkomplexen ausgewählt sind, bevorzugt. Erfindungsgemäß bevorzugte Schwermetallverbindungen sind ausgewählt aus der Gruppe bestehend aus Kupfer(II)-hydroxid, Kupfer(II)methacrylat, Kupfer(II)acetylacetonat, Kupfer(II)-2-ethyl-hexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon.

Gemäß einer anderen Ausgestaltungsform der erfindungsgemäßen Zusammensetzung oder Paste ist der Polymerisationsbeschleuniger ausgewählt aus der Gruppe bestehend aus N,N-Dimethyl-p-toluidin, N,N-Bis-hydroxyethyl-p-toluidin, N,N-Dimethyl-anilin, Trioctylmethyl-ammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid, Pyromelithsäurediimid und einer Mischung aus mindestens zwei davon.

Eine weitere vorteilhafte Ausgestaltung der Erfindung umfasst eine Verwendung von Kombinationen aus Schwermetallsalzen und mindestens einem Vertreter aus der Gruppe umfassend aus N,N-Dimethyl-p-toluidin, N,N-Bis-hydroxyethyl-p-toluidin, N,N-Dimethyl-anilin, Trioctylmethyl-ammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid und Pyromelithsäurediimid als Polymerisationsbeschleuniger. Dabei werden Zweifachkombinationen und auch Dreifachkombinationen von unterschiedlichen Polymerisationsbeschleunigern im Rahmen der Erfindung offenbart.

Eine vorteilhafte Ausgestaltung der Erfindung besteht darin, dass gegebenenfalls wenigstens ein Co-Polymerisationsbeschleuniger in der erfindungsgemäßen Zusammensetzung oder in einer der Pasten A, B oder der Monomerkomponente D enthalten ist, wobei als Co-Polymerisationsbeschleuniger tertiäre Amine und Amidine bevorzugt sind, und wobei ganz besonders N,N-Dimethyl-p-toluidin, N,N-Bis-hydroxyethyl-p-toluidin, N,N-Dimethyl-anilin, 1,8-Diazabicyclo[5.4.0-]undec-7-en und 1,5-Diazabicyclo(4.3.0)-non-5-en als Co-Akzeleratoren bevorzugt sind.

Die erfindungsgemäße Zusammensetzung, insbesondere in einer Paste oder Monomerkomponente kann den Polymerisationsinitiator, den Polymerisationsbeschleuniger, den Co-Polymerisationsbeschleuniger oder den Polymerisationsinitiator, den Polymerisationsbeschleuniger und den Co-Polymerisationsbeschleuniger in einer (Gesamt)Menge von bis zu 10 Gew.-%, bezogen auf das Gesamtgewicht der erfindungsgemäßen Zusammensetzung oder jeweils unabhängig bezogen auf das Gesamtgewicht einer der Pasten A, B oder der Monomerkomponente D beinhalten.

Die erfindungsgemäße Zusammensetzung und insbesondere die Pasten A und B als auch Monomerkomponente D und Pulverkomponente C können neben den vorstehend genannten Komponenten weitere Bestandteile beinhalten.

Gemäß einer bevorzugten Ausführungsform der erfindungsgemäßen Zusammensetzung oder einer der Pasten A oder B sowie der Monomerkomponente D oder der Pulverkomponente C können diese jeweils unabhängig voneinander mindestens einen Röntgenopaker beinhalten.

Beim Röntgenopaker kann es sich um einen fachüblichen Röntgenopaker handeln. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen besteht. Diese Röntgenopaker weisen vorzugsweise einen mittle-ren Teilchendurchmesser im Bereich von 10 nm bis 500 $\mu$m auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht. Die Konzentrationen an Röntgenopaker, insbesondere die Konzentration an Zirkoniumdioxid, in der erfindungsgemäßen Zusammensetzung oder einer der Pasten A oder B sowie in der Pulverkomponente C oder der Monomerkomponente D können jeweils voneinander unabhängig beispielsweise in einem Bereich von 3 bis 30 Gew.-% in Bezug auf die jeweilige Gesamtzusammensetzung liegen. Röntgenopaker gelten vorliegend nicht als Füllstoffe.

Gemäß einer weiteren bevorzugten Ausführungsform kann die erfindungsgemäße Zusammensetzung oder eine der vorgenannten Pasten mindestens einen Farbstoff beinhalten. Der Farbstoff kann ein fachüblicher Farbstoff und vorzugsweise ein Lebensmittelfarbstoff sein. Ferner kann der Farbstoff in dem wenigstens einem radikalisch polymerisierbaren Monomer löslich oder unlöslich sein. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132.

Gemäß einer weiteren bevorzugten Ausführungsform kann die erfindungsgemäße Zusammensetzung mindestens ein biokompatibles Elastomer beinhalten. Das biokompatible Elastomer ist vorzugsweise partikulär. Vorzugsweise ist das biokompatible Elastomer in dem wenigstens einen radikalisch polymerisierbaren Monomer löslich. Als besonders geeignet hat sich die Verwendung von Polybutadien als biokompatibles Elastomer erwiesen.

Gemäß einer weiteren bevorzugten Ausführungsform kann die erfindungsgemäße Zusammensetzung mindestens ein Monomer mit Haftgruppen beinhalten. Bei der Haftgruppe kann es sich beispielsweise um eine Amidgruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Gemäß einer weiteren bevorzugten Ausführungsform kann die erfindungsgemäße Zusammensetzung oder mindestens eine der Pasten A oder B oder die Monomerkomponente D mindestens einen Polymerisationsinhibitor, auch Stabilisator, beinhalten. Der Polymerisationsinhibitor soll geeignet sein, eine Spontanpolymerisation der in der Paste enthaltenen radikalisch polymerisierbaren Monomere zu verhindern. Ferner soll der Polymerisationsinhibitor keine störenden Wechselwirkungen mit den anderen in der erfindungsgemäßen Paste enthaltenen Bestandteilen zeigen. Derartige Polymerisationsinhibitoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butyl-phenol.

Die Erfindung wird durch nachstehende Beispiele näher erläutert, ohne jedoch die Erfindung zu beschränken.

Beispiele 1-5

Bestimmung der mechanischen Parameter nach ISO5833

Für die Herstellung der nachfolgenden tabellarisch dargestellten Zementpulver wurde 75%iges Dibenzoylperoxid als Polymerisationsinitiator (BPO, phlegmatisiert mit 25 Gew.-% Wasser, bezogen von Akzo Nobel, Charge Nr. 2612211601), Magnesiummonoperoxy-o-phthalat-Hexahydrat als Monoperoxydicarbonsäure-Salz (technical grade, Gehalt ca. 80 %, bezogen von Sigma Aldrich, Produkt Nr. 69868, Partikelgröße < 250 $\mu$m), Zirkoniumdioxid als Röntgenopaker (bezogen von S. Goldmann, Charge Nr. FB100856) und Poly(methylmethacrylat-co-methylacrylat) (PMMA-co-MA, bezogen von Evonik, Charge Nr. 310HDF129) als Copolymer-Komponente verwendet.

| Beispiel Nr. | Zusammensetzung / g | | | | |
|---|---|---|---|---|---|
| | Magnesiummonoperoxy-o-phthalat (MMPP) | | PMM-co-MA (Copolymer) | Zirkoniumdioxid | BPO |
| | MMPPx6H$_2$O (Hexahydrat) | MMPP (Reinsubstanz) | | | |
| 1 (Vergleichsbeispiel) | - | - | 33,41 | 6,0 | 0,59 |
| 2 | 0,63 | 0,50 | 33,41 | 6,0 | 0,59 |
| 3 | 1,25 | 1,00 | 33,41 | 6,0 | 0,59 |
| 4 | 1,88 | 1,50 | 33,41 | 6,0 | 0,59 |
| 5 | 2,50 | 2,00 | 33,41 | 6,0 | 0,59 |

Beispiel 1 ist ein Vergleichsbeispiel ohne Monoperoxydicarbonsäure-Salz. Die Beispiele 2 bis 5 sind Beispiele gemäß der Erfindung.

Entsprechend der oben genannten Zusammensetzungen wurden in Beispiel 1 (Vergleichsbeispiel) 40 g Pulverkomponente eingesetzt, in Beispiel 2 40,63 g Pulverkomponente eingesetzt, in Beispiel 3 41,25 g Pulverkomponente eingesetzt, in Beispiel 4 43,38 g Pulverkomponente eingesetzt und in Beispiel 5 44,5 g Pulverkomponente eingesetzt.

Für die Herstellung von Zementteig zur Bestimmung der mechanischen Eigenschaften und der antimikrobiellen Wirksamkeit wurde eine flüssige Monomerkomponente (Heraeus Medical, Charge Nr. 5271) mit folgender Zusammensetzung verwendet: 98,0 Gew.-% Methylmethacrylat als radikalisch polymerisierbares Monomer, 2,0 Gew.-% N,N-Dimethyl-p-toluidin als Polymerisationsbeschleuniger und 20 ppm p-Hydrochinon als Polymerisationsinhibitor.

Herstellung von Prüfkörpern

Die ISO 5833 fordert eine Biegefestigkeit von $\geq$ 50 MPa, ein Biegemodul von $\geq$ 1800 MPa und eine Druckfestigkeit von $\geq$ 70 MPa. Zur Prüfung der mechanischen Eigenschaften gemäß der ISO 5833 wurden Prüfkörper hergestellt.

Dafür wurden die Zementpulver gemäß der oben genannten Zusammensetzungen der Beispiele 1 bis 5 mit jeweils 20 ml flüssiger Monomerkomponente vermischt. Es entstand nach ca. 60 Sekunden ein klebfreier, plastisch verformbarer, viskoser Zementteig, der nach wenigen Minuten aushärtete. Es wurden mit dem Zementteig des Vergleichsbeispiels 1 und der erfindungsgemäßen Beispiele 2 bis 5 jeweils streifenförmige Probekörper der Abmessungen 75 mm x 10 mm x 3,3 mm für die Prüfung der Biegefestigkeit und des Biegemoduls gemäß ISO5833 hergestellt. Weiterhin wurden zylinderförmige Probekörper (Durchmesser 6 mm, Höhe 12 mm) für die Prüfung der Druckfestigkeit gefertigt.

Nach Lagerung der Probekörper bei 23 °C, einer relativen Luftfeuchtigkeit von 50 %, über einen Zeitraum von 24 Stunden, wurden die Biegefestigkeit, das Biegemodul und die Druckfestigkeit gemäß ISO 5833 mit Hilfe einer Zwick-Universal-Prüfmaschine bestimmt. Die Ergebnisse sind in der nachfolgenden Tabelle zusammengefasst.

| Beispiel Nr. | Biegefestigkeit [MPa] | Biegemodul [MPa] | Druckfestigkeit [MPa] |
|---|---|---|---|
| | Anforderung nach ISO 5833 | | |
| | $\geq$ 50 MPa | $\geq$ 1800 MPa | $\geq$ 70 MPa |
| 1 | 72,1 $\pm$ 1,5 | 2832 $\pm$ 43 | 99,7 $\pm$ 1,1 |

(fortgesetzt)

| Beispiel Nr. | Biegefestigkeit [MPa] | Biegemodul [MPa] | Druckfestigkeit [MPa] |
|---|---|---|---|
| | Anforderung nach ISO 5833 | | |
| | $\geq$ 50 MPa | $\geq$ 1800 MPa | $\geq$ 70 MPa |
| 2 | 72,2 $\pm$ 3,1 | 2832 $\pm$ 31 | 100,3 $\pm$ 1,5 |
| 3 | 68,6 $\pm$ 2,9 | 2735 $\pm$ 46 | 99,4 $\pm$ 1,9 |
| 4 | 70,1 $\pm$ 2,7 | 2704 $\pm$ 30 | 98,8 $\pm$ 1,0 |
| 5 | 67,1 $\pm$ 2,7 | 2686 $\pm$ 47 | 99,4 $\pm$1,4 |

Die Ergebnisse zeigen, dass die mechanischen Anforderungen der ISO5833 bezüglich der Biegefestigkeit, des Biegemoduls und der Druckfestigkeit von den Zementen sowohl im Vergleichsbeispiel 1 als auch in den erfindungsgemäßen Beispielen 2 bis 5 erfüllt wurden. Es wurde weiterhin gezeigt, dass der Zusatz des Monoperoxydicarbonsäure-Salzes keinen negativen Einfluss auf die radikalische Polymerisation hatte und dadurch in den Beispielen 2 bis 5 Prüfkörper mit vergleichbaren mechanischen Eigenschaften erhalten werden.

Bestimmung der antimikrobiellen Wirksamkeit in Gegenwart von defibrinierten Schafblut

Die Prüfung der antimikrobiellen Wirksamkeit erfolgte mit der Filmkontaktmethode in Anlehnung an JIS Z 2801 (Japanischer Industriestandard). Es wurden von den Zementen des Vergleichsbeispiels 1 und der erfindungsgemäßen Beispiele 2 bis 5 streifenförmige Zementprüfkörper mit den Abmessungen 50 mm x 50 mm x 3 mm hergestellt, jeweils 3 Prüfkörper von jedem Beispiel. Die Prüfkörper wurden oberflächlich mit einer 70 Gew.-%igen wässrigen Ethanollösung desinfiziert. Als Testkeim wurde *Staphylococcus aureus* ATCC6538 verwendet. Der Testkeim wurde in 5 Gew.-%iger Blutlösung (5 Gew.-% defibriniertes Schafblut in 0,9%iger wässriger Natriumchlorid-Lösung) inokuliert. Es wurden jeweils 0,2 ml einer Suspension des Testkeims mit einer Konzentration von 05,-2,0 x $10^6$ KBE/ml auf die Prüfkörperoberfläche aufgetragen. Daraus resultierte eine Keimbelastung von 1,0-4,0 x $10^5$ KBE/ml. Auf die Keimsuspension wurde eine Kunststofffolie gelegt, so dass der Abstand der Prüfkörperoberfläche zur Kunststofffolie ca. 100 $\mu$m betrug. Die beimpften Prüfkörper wurden in einer Wasserdampf-gesättigten Atmosphäre bei 36 $\pm$ 1 °C für 24 Stunden bebrütet. Anschließend erfolgte die Ablösung der Keime in PE-Beuteln mit jeweils 10 ml physiologischer Kochsalzlösung. Die Keimsuspensionen wurden auf TSA-Platten (Trypticase Soja Agar) ausplattiert. Die TSA-Platten wurden dann für 40-48 Stunden bei 36 $\pm$ 1 °C inkubiert. Danach erfolgte die Auszählung der entstandenen Kolonien mit einem Colony Counter. Unter Berücksichtigung der Verdünnung wurden die Keimzahlen pro Prüfkörper bestimmt. Es wurden von jeweils drei Prüfkörpern jedes Beispiels die Mittelwerte der Keimzahlen gebildet und unter Berücksichtigung der Referenzproben der Reduktionsfaktor berechnet.

$$\text{Reduktionsfaktor (RF)} = c\text{-}d$$

wobei

c: Arithmetisches Mittel der $\log_{10}$-Keimzahlen auf den inkubierten Probekörperoberflächen

d: Arithmetisches Mittel der $\log_{10}$-Keimzahlen auf den inkubierten Referenzprobekörperoberflächen

| Beispiel Nr. | Reduktionsfaktor |
|---|---|
| 1 (Vergleichsbeispiel) | Keine Reduktion |
| 2 | 4,32 |
| 3 | > 4,99 |
| 4 | > 4,99 |
| 5 | > 4,99 |

Die Zemente der Beispiele 2-5 zeigen eine signifikante Reduktion der Keimzahlen um 4 Logstufen. Das bedeutet, dass mindestens 99,99 % der Testkeime abgetötet wurden.

Bevorzugte Ausgestaltungen von Bestandteilen einer erfindungsgemäßen Kategorie sind ebenso bevorzugt für gleichnamige oder entsprechende Bestandteile der jeweils anderen erfindungsgemäßen Kategorie. Die Ausdrücke "aufweisend", "umfassend" oder "beinhaltend" usw. schließen nicht aus, dass weitere Elemente, Inhaltsstoffe usw. enthalten sein können. Der unbestimmte Artikel "ein" schließt nicht aus, dass eine Mehrzahl vorhanden sein kann.

**Patentansprüche**

1. Zusammensetzung zur Verwendung als Knochenzement, wobei die Zusammensetzung härtbar ist, **dadurch gekennzeichnet, dass** die Zusammensetzung ein pharmakologisch verträgliches Salz einer Monoperoxydicarbonsäure aufweist und das Salz der Monoperoxydicarbonsäure in Gegenwart von Wasser aus der Zusammensetzung herauslösbar ist.

2. Zusammensetzung nach Anspruch 1, **dadurch gekennzeichnet, dass** die Zusammensetzung ein antiseptischer Knochenzement ist.

3. Zusammensetzung nach einem der Ansprüche 1 oder 2, **dadurch gekennzeichnet, dass** die Zusammensetzung ein antiseptischer Polymethylmethacrylat-Knochenzement ist.

4. Zusammensetzung nach einem der Ansprüche 1 bis 3, **dadurch gekennzeichnet, dass** das Salz der Monoperoxydicarbonsäure ein Erdalkalisalz oder ein Alkalisalz ist.

5. Zusammensetzung nach einem der Ansprüche 1 bis 4, **dadurch gekennzeichnet, dass** das Erdalkalisalz eine Magnesiumsalz ist.

6. Zusammensetzung nach einem der Ansprüche 1 bis 5, **dadurch gekennzeichnet, dass** das Salz der Monoperoxydicarbonsäure in Methylmethacrylat bei Raumtemperatur nicht löslich ist.

7. Zusammensetzung nach einem der Ansprüche 1 bis 6, **dadurch gekennzeichnet, dass** das Salz der Monoperoxydicarbonsäure in der Zusammensetzung als Pulver eingesetzt wird, wobei das Pulver eine mittlere Partikelgröße von nicht mehr als 250 $\mu$m aufweist.

8. Zusammensetzung nach einem der Ansprüche 1 bis 7, **dadurch gekennzeichnet, dass** das Salz der Monoperoxydicarbonsäure in wässriger Lösung bei Raumtemperatur innerhalb von 5 min nicht durch das Enzym Katalase zersetzt wird.

9. Zusammensetzung nach einem der Ansprüche 1 bis 8, **dadurch gekennzeichnet, dass** das Salz der Monoperoxydicarbonsäure zu 0,5 Gew.-% bis 6,0 Gew.-% bezogen auf die Gesamtmenge der Zusammensetzung enthalten ist.

10. Zusammensetzung nach einem der Ansprüche 1 bis 9, **dadurch gekennzeichnet, dass** die Monoperoxydicarbonsäure ausgewählt ist aus mindestens einem Element der Gruppe Monoperoxyphthalsäure, Monoperoxyglutarsäure, Monoperoxybernsteinsäure und Monoperoxycyclohexyldicarbonsäure.

11. Zusammensetzung nach Anspruch 10, **dadurch gekennzeichnet, dass** die Monoperoxydicarbonsäure Monoperoxyphthalsäure ist.

12. Zusammensetzung nach einem der Ansprüche 10 oder 11, **dadurch gekennzeichnet, dass** die Zusammensetzung das Magnesiumsalz der Monoperoxyphthalsäure aufweist.

13. Zusammensetzung nach einem der Ansprüche 1 bis 12, **dadurch gekennzeichnet, dass** die Zusammensetzung mindestens ein radikalisch polymerisierbares Monomer und mindestens ein organisches Polymer umfasst, wobei das Polymer in dem Monomer löslich ist.

14. Zusammensetzung nach einem der Ansprüche 1 bis 13, **dadurch gekennzeichnet, dass** das organische Polymer ausgewählt ist aus Poly(alkyl-2-acrylsäure-alkylester), Poly(aryl-2-acrylsäurealkylester), Poly(arylalkyl-2-acrylsäure-alkylester) jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Polymere.

15. Zusammensetzung nach einem der Ansprüche 13 oder 14, **dadurch gekennzeichnet, dass** das organische Polymer ausgewählt ist aus der Gruppe Poly(methacrylsäuremethylester), Poly(methacrylsäureethylester), Poly(methylmethacrylsäurepropylester), Poly(methacrylsäureisopropylester), Poly(methylmethacrylat-co-methylacrylat), Poly(styrol-co-methylmethacrylat), Copolymeren der genannten Verbindungen und einer Mischung umfassend mindestens zwei dieser Polymere.

16. Zusammensetzung nach einem der Ansprüche 12 bis 15, **dadurch gekennzeichnet, dass** das Monomer ausgewählt ist aus mindestens einem 2-Alkyl-acrylsäurealkylester, 2-Arylacrylsäurealkylester, 2-Arylalkyl-acrylsäurealkylester jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Monomere.

17. Zusammensetzung nach einem der Ansprüche 13 bis 16, **dadurch gekennzeichnet, dass** das organische Polymer mindestens ein Poly(methacrylsäuremethylester), (PMMA) oder ein Poly(methacrylsäuremethylester-copolymer) und als Monomer Methacrylsäuremethylester (MMA) umfasst.

18. Kit aufweisend eine Paste A und eine Paste B, wobei

   (a) Paste A

   (a1) mindestens ein radikalisch polymerisierbares Monomer,
   (a2) mindestens ein in (a1) lösliches organisches Polymer,
   (a3) optional mindestens einen Polymerisationsinhibitor und
   (a4) mindestens eine Komponente eines Redoxinitiatorsystems beinhaltet;

   (b) Paste B

   (b1) mindestens ein radikalisch polymerisierbares Monomer,
   (b2) mindestens ein in (b1) lösliches organisches Polymer, und
   (b3) mindestens einen Polymerisationsbeschleuniger beinhaltet;

   und wobei mindestens eine der Pasten A und/oder B als Komponente (a5) beziehungsweise (b4) ein pharmakologisch verträgliches Salz einer Monoperoxydicarbonsäure beinhaltet.

19. Kit aufweisend eine Pulverkomponente C und eine flüssige Monomerkomponente D, wobei die

   (c) Pulverkomponente C

   (c1) mindestens ein pulverförmiges Poly(meth)acrylat,
   (c2) mindestens einen pulverförmigen Röntgenopaker,
   (c3) mindestens einen Polymerisationsinitiator beinhaltet;

   (d) Monomerkomponente D

   (d1) wenigstens ein radikalisch polymerisierbares Monomer,
   (d2) optional wenigstens einen Polymerisationsinhibitor,
   (d3) optional wenigstens ein in (d1) lösliches organisches Polymer und
   (d4) wenigstens einen Polymerisationsbeschleuniger beinhaltet;

   und wobei die Pulverkomponente C als Komponente (c4) ein pharmakologisch verträgliches Salz einer Monoperoxydicarbonsäure beinhaltet.

20. Härtbarer Knochenzement, erhältlich durch Polymersation einer Zusammensetzung nach einem der Ansprüche 1 bis 17 oder durch Vermischen der Pasten A und B nach Anspruch 18 oder durch Vermischen der Pulverkomponente C mit der Monomerkomponente D nach Anspruch 19.

21. Gehärteter Knochenzement, erhältlich durch Polymerisation einer Zusammensetzung nach einem der Ansprüche 1 bis 17, oder erhältlich durch Vermischen und Polymerisation der Pasten A und B oder der Pulverkomponente C mit der Monomerkomponente D nach einem der Ansprüche 18 oder 19, wobei der Gehalt des pharmakologisch verträglichen Salzes der Monoperoxydicarbonsäure im gehärteten Knochenzement 0,5 Gew.-% bis 6,0 Gew.-% in Bezug auf die Gesamtzusammensetzung beträgt.

22. Gehärteter Knochenzement nach Anspruch 21, **dadurch gekennzeichnet, dass** das Salz der Monoperoxydicarbonsäure bei der Aushärtung in Gegenwart von Wasser aus der Zusammensetzung herausgelöst wird und die Monoperoxydicarbonsäure innerhalb von 5 min nicht durch das Enzym Katalase zersetzt wird.

23. Formkörper erhältlich durch Polymerisation einer Zusammensetzung nach einem der Ansprüche 1 bis 17 oder durch Vermischen der Pasten A und B nach Anspruch 18 oder durch Vermischen der Pulverkomponente C mit der Monomerkomponente D nach Anspruch 19 und anschließender Polymerisation.

24. Verwendung einer Zusammensetzung nach einem der Ansprüche 1 bis 17 oder eines Kits nach Anspruch 18 oder 19 als Implantat, als antiseptisches Implantat, als Revisionsimplantat, zur mechanischen Fixierung primärer Totalgelenkendoprothesen, zur mechanischen Fixierung von Revisions-Totalgelenkendoprothesen, zur Augmentation von osteoporotischem Knochengewebe und ganz besonders bevorzugt für die Vertebroplastie, Kyphoplastie und die Augmentation von Bohrlöchern in osteoporotischem Knochengewebe, zur Auffüllung von Knochenkavitäten, zur Femuroplastie, zur Herstellung von Spacern, zur mechanischen Fixierung von Gelenkprothesen, zur Abdeckung von Schädeldefekten oder zur Herstellung von Trägermaterialien für die lokale Antibiotika-Therapie oder als Trägermaterial für eine lokale Freisetzung von pharmazeutisch wirksamen Substanzen.

**Europäisches Patentamt**
**European Patent Office**
**Office européen des brevets**

# EUROPÄISCHER RECHERCHENBERICHT

Nummer der Anmeldung

EP 17 16 7025

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | DE 10 2012 022419 A1 (HERAEUS MEDICAL GMBH [DE]) 22. Mai 2014 (2014-05-22) * Seite 9 - Seite 10; Ansprüche 1,5,16-31 * * Absatz [0100] - Absatz [0104] * ----- | 1-24 | INV. A61L24/00 A61K33/40 C08L33/12 |
| X | DE 195 08 827 A1 (BODE CHEMIE GMBH & CO [DE]) 12. September 1996 (1996-09-12) * Spalte 1 - Spalte 2; Ansprüche 1,5,6; Beispiel 1 * ----- | 1-24 | |
| X | DE 10 2009 005534 B3 (HERAEUS MEDICAL GMBH [DE]) 1. April 2010 (2010-04-01) * Absatz [0009]; Ansprüche 1,6,7; Beispiele * ----- | 1-24 | |
| A | DE 10 2014 105267 A1 (HERAEUS MEDICAL GMBH [DE]) 15. Oktober 2015 (2015-10-15) * Seite 7 - Seite 8 * ----- | 1-24 | |
| X | DE 198 08 962 C2 (BODE CHEMIE GMBH & CO KG [DE]) 28. März 2002 (2002-03-28) * Seite 3, Absatz 0007; Tabellen * ----- | 1-24 | RECHERCHIERTE SACHGEBIETE (IPC) A61L A61K C08L |
| A | DE 37 30 921 A1 (DEGUSSA [DE]) 23. März 1989 (1989-03-23) * Seite 2 - Seite 3.; Anspruch 1 * ----- | 1-24 | |

Der vorliegende Recherchenbericht wurde für alle Patentansprüche erstellt

| Recherchenort | Abschlußdatum der Recherche | Prüfer |
|---|---|---|
| München | 7. August 2017 | Härtinger, Stefan |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
    Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 17 16 7025

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-08-2017

| Im Recherchenbericht angeführtes Patentdokument | Datum der Veröffentlichung | Mitglied(er) der Patentfamilie | Datum der Veröffentlichung |
|---|---|---|---|
| DE 102012022419 A1 | 22-05-2014 | AU 2013251224 A1<br>CA 2830573 A1<br>CN 103816566 A<br>DE 102012022419 A1<br>EP 2732830 A1<br>JP 2014100559 A<br>JP 2016154874 A<br>US 2014141097 A1 | 05-06-2014<br>16-05-2014<br>28-05-2014<br>22-05-2014<br>21-05-2014<br>05-06-2014<br>01-09-2016<br>22-05-2014 |
| DE 19508827 A1 | 12-09-1996 | AT 252898 T<br>CZ 9702847 A3<br>DE 19508827 A1<br>EP 0824347 A1<br>HU 9800715 A2<br>WO 9628147 A1 | 15-11-2003<br>14-01-1998<br>12-09-1996<br>25-02-1998<br>28-07-1998<br>19-09-1996 |
| DE 102009005534 B3 | 01-04-2010 | DE 102009005534 B3<br>EP 2198893 A2<br>JP 5661275 B2<br>JP 2010144168 A<br>US 2010159027 A1 | 01-04-2010<br>23-06-2010<br>28-01-2015<br>01-07-2010<br>24-06-2010 |
| DE 102014105267 A1 | 15-10-2015 | AU 2015201574 A1<br>CA 2885754 A1<br>CN 104971379 A<br>DE 102014105267 A1<br>EP 2932987 A1<br>JP 6010173 B2<br>JP 2015226755 A<br>US 2015290355 A1 | 29-10-2015<br>14-10-2015<br>14-10-2015<br>15-10-2015<br>21-10-2015<br>19-10-2016<br>17-12-2015<br>15-10-2015 |
| DE 19808962 C2 | 28-03-2002 | DE 19808962 A1<br>EP 0940081 A1 | 09-12-1999<br>08-09-1999 |
| DE 3730921 A1 | 23-03-1989 | DE 3730921 A1<br>EP 0308359 A1<br>IL 87375 A<br>JP H01113405 A<br>US 4866146 A | 23-03-1989<br>22-03-1989<br>15-01-1992<br>02-05-1989<br>12-09-1989 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- DE 102007052116 A1
- DE 3245956 A1
- DE 102007050762 B3
- DE 102008030312 A1
- WO 8201990 A1
- US 4849223 A
- EP 1313518 A1
- EP 1648531 A1
- DE 102012022419 A1
- DE 102010024653 B4
- DE 102010005956 B4

**In der Beschreibung aufgeführte Nicht-Patentliteratur**

- **K.-D. KÜHN.** PMMA Cements. Springer-Verlag Berlin Heidelberg, 2014